## Europäisches Patentamt
## European Patent Office
## Office européen des brevets

(19)

(11) Veröffentlichungsnummer: **0 089 035**
**B1**

(12) # EUROPÄISCHE PATENTSCHRIFT

(45) Veröffentlichungstag der Patentschrift:
**16.09.87**

(51) Int. Cl.⁴: **A 61 F 5/47**

(21) Anmeldenummer: **83102445.0**

(22) Anmeldetag: **12.03.83**

(54) **Tubenpessar.**

(30) Priorität: **15.03.82 FR 8204303**

(43) Veröffentlichungstag der Anmeldung:
**21.09.83 Patentblatt 83/38**

(45) Bekanntmachung des Hinweises auf die Patenterteilung:
**16.09.87 Patentblatt 87/38**

(84) Benannte Vertragsstaaten:
**AT BE CH DE FR GB IT LI NL SE**

(56) Entgegenhaltungen:
**EP - A - 0 010 812**
**WO - A - 81/01515**
**DE - A - 2 328 175**
**DE - A - 2 913 036**
**FR - A - 2 427 825**
**GB - A - 1 568 419**
**US - A - 3 515 132**
**US - A - 3 858 571**
**US - A - 3 935 860**
**US - A - 4 004 582**

(73) Patentinhaber: **MED-Inventio AG, Seestrasse 359, CH-8038 Zürich-Wollishofen (CH)**

(72) Erfinder: **Hamou, Jacques, 2, Chaussee de la Muette, F-75016 Paris (FR)**

(74) Vertreter: **Wenzel, Joachim, Dipl.-Ing., Hauptmannsreute 46, D-7000 Stuttgart 1 (DE)**

**Beschreibung**

Die Erfindung betrifft ein Tubenpessar zum beidseitigen reversiblen Verschliessen des isthmischen Teils der Tube der Gebärmutter mit einem zylindrischen Mittelstück aus medizinisch unwirksamen Material und Ansätzen an beiden Enden des Mittelstückes, wobei die Ansätze an dem distalen Ende nach aussen federnd sich spreizend angeordnet sind.

Es ist bereits ein Tuben-Occlusivpessar zum reversiblen Verschliessen von Tuben innerhalb des menschlichen oder tierischen Organismus vom Uterus her bekannt. Dabei ist vorgesehen, dass der isthmische Kanal verschlossen wird, der zwischen dem Cervix und dem Uterus liegt. Hierbei zeigt das bekannte Tubenpessar einen nach hinten sich konisch stark erweiternden Teil mit abgerundeten Enden, dessen grösster Durchmesser 6 mm betragen muss, um sich dem Cervix-Kanal an dieser Stelle anzupassen und einen absolut dichten Abschluss der Tube anzustreben. Das distale Ende des bekannten Tuben-Occlusivpessares zeigt zum proximalen Ende hin gerichtete elastische Ringscheiben für das Festhalten des Pessares in der Tube und kleinere elastische Ringscheiben im Mittelbereich des Pessares für das Abdichten der Tube im interstitiellen Teil (DE-A 2 382 175, Fig. 4).

Es gibt auch ein Mittel zum Verschliessen der trompetenförmigen Erweiterungen durch Injektion mittels eines Hysteroskopes, wobei eine Mischung aus einem flüssigen Vor-Elastomer und einem Katalysator verwendet wird, der zur Polimerisation des Materials in den Eileiter eingespritzt wird. Aber dieses Verfahren erfordert eine besondere Technik mit lokaler Anästhesie, wozu eine Pumpvorrichtung zur Injektion erforderlich ist, ausserdem ergibt sich hierdurch die Gefahr des Misslingens und der erhöhten Risiken, was besonders durch die Unbeständigkeit des Injektionsmaterials verursacht wird. Die Umkehrbarkeit durch die Anwendung der Hysteroskopie ist theoretisch möglich, aber der Durchgang des distalen Aussenendes der Spritze mit einem Durchmesser von mehreren Millimetern kann den isthmischen Kanal der trompetenförmigen Erweiterung mit einem Durchmesser unterhalb 1 mm verletzen, und zwar trotz der Elastizität des Elastomers.

Der Erfindung liegt die Aufgabe zugrunde, das Tubenpessar der erwähnten Art so zu verbessern, dass es zum beidseitigen reversiblen Verschliessen des isthmischen Teils der Tube der Gebärmutter geeignet ist. Der isthmische Kanal zwischen dem Uterus und der Tube hat einen Durchmesser von 0,4–1 mm, wodurch das Tubenpessar wesentlich kleiner ausfallen kann. Auf diese Weise besteht darüberhinaus die Möglichkeit, das Tubenpessar innerhalb des Instrumentenkanals eines Hysteroskopes unterzubringen, während bisher erforderlich gewesen ist, diesen aussen auf das Instrument aufzusetzen infolge der erwähnten grossen Stärke von 6 mm.

Derartige Hysteroskope sind bereits auf dem Gebiet der Gynäkologie bekannt, andererseits besteht aber auch die Möglichkeit, eine Klammer zu verwenden.

Zur Lösung dieser Aufgabe sind die kennzeichnenden Merkmale des Anspruchs 1 vorgesehen. Dadurch wird die Aufgabe gelöst, und der Erfindungsgegenstand lässt sich verhältnismässig leicht in den isthmischen Kanal einbringen, weil die Ansätze der beiden Enden fadenförmig ausgebildet sind.

Weitere Vorteile und Einzelheiten der Erfindung ergeben sich aus der nun folgenden Beschreibung einiger Ausführungsbeispiele unter Hinweis auf die Zeichnung. In dieser zeigen:

Fig. 1 eine schematische erste Ausführungsform des erfindungsgemässen Tubenpessars;

Fig. 2 das Tubenpessar nach Fig. 1 in seiner Wirklage;

Fig. 3 eine weitere Ausführungsform;

Fig. 4 eine Ansicht nur auf das distale Ende einer weiteren Ausführungsform;

Fig. 5 einen teilweisen Schnitt durch den Kanal mit dem distalen Ende der Vorrichtung nach Fig. 4;

Fig. 6 eine perspektivische Ansicht auf das distale Ende einer weiteren Ausführungsform und

Fig. 7 einen Schnitt durch den Kanal 7 eines Hysteroskopes mit dem distalen Ende nach der Fig. 6.

Das Mittelstück 1 des Tubenpessars zeigt einen zylindrischen Querschnitt, ist weich und aus plastischem Material wie Nylon mit medizinischer Qualität und medizinisch unwirksam. Die Ausführungsform ist etwa 1 cm lang und hat einen Durchmesser von 1–2 mm, aber es können auch andere Abmessungen vorgesehen sein, um sich der Funktion und der Anatomie des isthmischen Teils der Tube anzupassen. Sein distales Aussenende 3 soll weich und abgerundet sein, um alle Verletzungen in diesem Bereich zu vermeiden. Der proximale Bereich 4 kann erweitert sein, um sich der Trichterform des Eingangs der trichterförmigen Erweiterung anzupassen.

Die distale Schleife 2 kann vorzugsweise aus chirurgischem Nylon oder einem anderen weichen Material bestehen, das medizinisch unwirksam ist. Bei einer Ausführungsform ist die Schleife oval und hat einen Durchmesser von 3–6 mm, wobei ein spitzer Winkel oder eine Abrundung das distale Ende 3 bildet, um ein leichtes Einführen zu ermöglichen. Dabei können die beiden Teile 6a, 6b leicht einander genähert werden, um sie in den Kanal 7 des Hysteroskopes 8 zu führen und sie dann in den isthmischen Teil 9 der Tube zu schieben. Anschliessend spreizen sich wieder diese Teile, nachdem sie sich in ihrer gewünschten Lage befinden, um eine ungewünschte Verschiebung des Tubenpessars 1 in Richtung der Gebärmutter zu verhindern.

Der Durchmesser dieser fadenförmigen in der erwähnten Weise geformten distalen Schleife 2 sollte vorzugsweise etwa die Hälfte des Durchmessers des Mittelstückes 1 betragen.

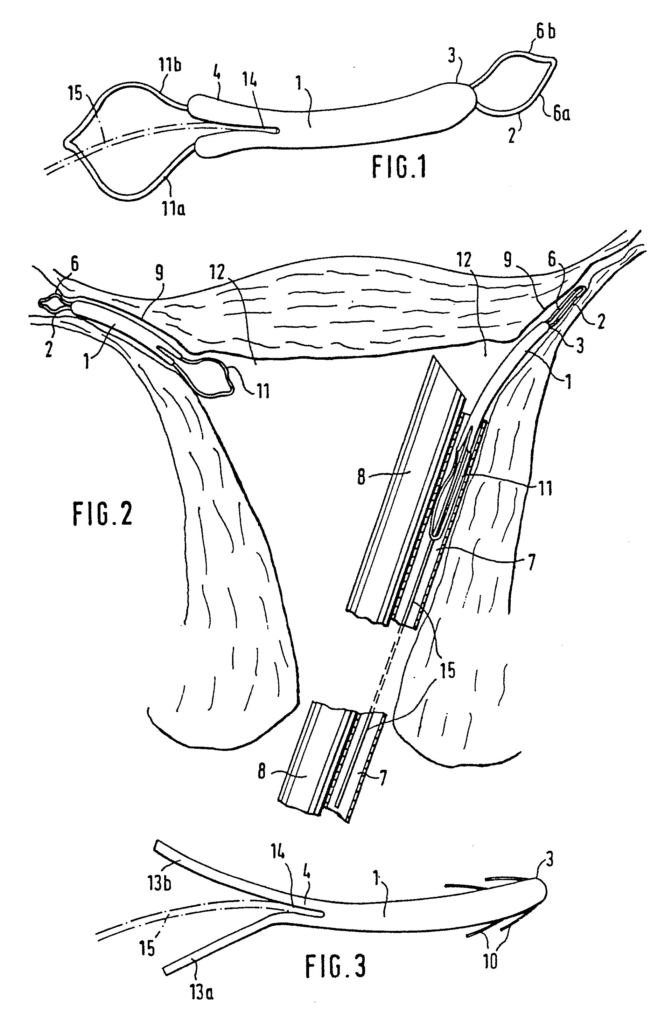

Gemäss der Fig. 3 kann das distale Ende 3 des Mittelstücks 1 mit mehreren kleinen Abzweigungen 10 versehen sein, die in die Richtung des proximalen Endes 4 weisen, um das Zurückgleiten zu verhindern und Krümmungen zur Anpassung an die Umgebung zu bilden.

Die proximale Schleife 11 nach der Fig. 1 soll bevorzugt ebenfalls so ähnlich wie die distale Schleife 2 ausgebildet sein, wobei die beiden Teile 11a, 11b ebenfalls in dem Kanal 7 des Hysteroskopes 8 aneinander genähert werden. In der Gebärmutter 12 spreizen diese Teile federnd auseinander und verhindern die Abwanderung des Tubenpessars in Richtung der Bauchhöhle. Die Schleife 11 dient anschliessend dem Rückzug der Vorrichtung durch das Hysteroskop mittels einer Zunge oder einer Klammer, die in dem Einführkanal 7 in bekannter Weise angeordnet ist.

Bei der weiteren Ausführungsform nach Fig. 3 können zur Abwanderung des Tubenpessars in Richtung der Bauchhöhle auch kleine Abzweigungen 13a, 13b elastisch vorgesehen sein, die ebenfalls eine gegenseitige Annäherung in dem Einführungskanal 7 ermöglichen, und die sich ebenfalls wieder in der Gebärmutter 12 voneinander spreizen, nachdem sie ihre Wirklage erreicht haben.

Bei einer weiteren vorteilhaften Ausführungsform kann auch ein Blindkanal 14 gem. Fig. 1 oder 3 im Bereich des proximalen Endes 4 vorgesehen sein, um die Einführung eines aus Metall bestehenden Führungsstückes 15 zu ermöglichen, welches flexibel ist und die Einführung und Richtungsgebung des Tubenpessars an Ort und Stelle erleichtert.

Gemäss einer weiteren bevorzugten Ausführungsform kann das Tubenpessar mit einem gegen Röntgenstrahlen undurchlässigen Mittel versehen werden, und zwar entweder durch einen axialen Zusatz, oder indem sich das Mittel in der Masse einer metallischen Substanz befindet, oder es kann sich auch um ein chemisches Mittel handeln, das eine gewisse Undurchlässigkeit gegen die Strahlung aufweist.

Bei einer weiteren Ausführungsform kann ein pharmakodynamischer, physikalischer oder chemischer Effekt gewünscht sein, wozu die Oberfläche der Vorrichtung mit chemisch-medikamentösen, metallischen Überzügen oder unveränderlichen Legierungen versehen wird.

Wie oben schon erwähnt, erfolgt die Einführung des Tubenpessars mittels eines Hysteroskopes. Hierzu wird das Tubenpessar in den Einführungskanal 7 des Endoskopes gegeben und mittels eines Führungsdrahtes 15 nach vorn gestossen, der aus Metall oder einem plastischen Material besteht, und zwar so weit, bis die Vorrichtung ihre Endlage erreicht hat. Dies ist in Fig. 2 dargestellt.

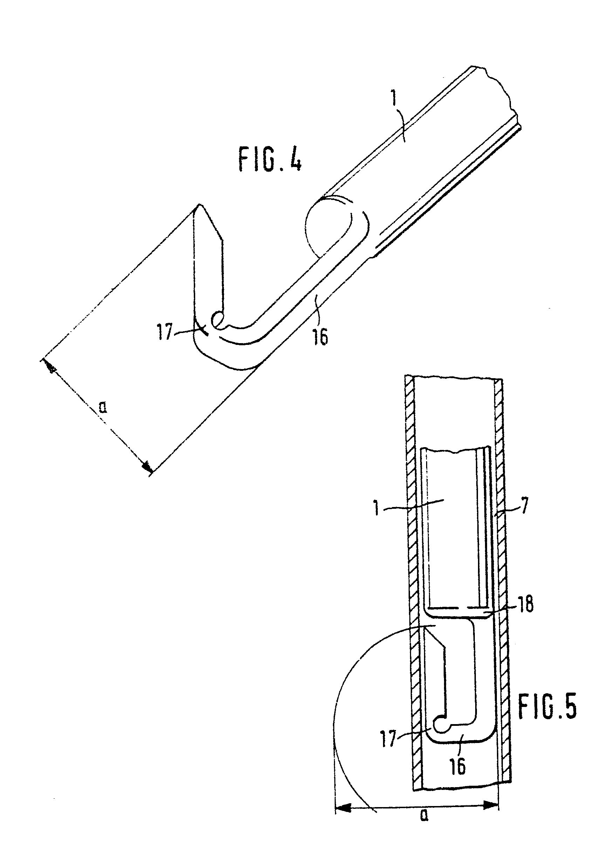

Fig. 4 zeigt eine perspektivische Ansicht des distalen Endes einer weiteren Ausführungsform. Hierbei ist das distale Ende als Widerhaken 16 ausgebildet, der exzentrisch zu dem Mittelstück 1 liegt, d.h., der Haken 16 bildet eine exzentrische Fortsetzung des Mittelstückes 1 zum distalen Ende hin, bevor der Haken die Biegung zum proximalen Ende hin zeigt. Wie man auch der Fig. 5 entnehmen kann, ist an der Stelle der stärksten Biegung des Widerhakens 16 eine Schwachstelle 17 angeordnet, deren Querschnitt somit kleiner ist als der des Hakens 16 im übrigen. Dadurch kann auch die Elastizität oder Federsteifigkeit des Hakens 16 beeinflusst werden. Fig. 4 zeigt die Ruhestellung ausserhalb des Kanals 7, bei der das Aussenende des scharfen Hakens 16 um das Mass a elastisch nach aussen gespreizt ist. Der Durchmesser des Mittelstückes 1 beträgt in diesem Falle 1 mm, somit ist der Durchmesser des Hakens 16 natürlich entsprechend kleiner.

Fig. 5 zeigt das distale Ende des Kanals des Hysteroskopes. Der am distalen Ende sitzende Haken 16 hat hier eine Gesamtlänge von 3 mm. Das Aussenende des Hakens 16 ist hier so stark federnd angewinkelt, dass der distale Haken 16 etwa U-förmig ausgebildet ist und das Mittelstück 1 mit Ausnahme der exzentrischen Verbindung 18 gar nicht berührt. Dies zeigt den Vorteil, dass der Haken 16 in dem Kanal 17, der hier eine lichte Weite von 1,2 mm aufweist, gut untergebracht werden kann. Hier ist auch wieder die proximale Spreizung a angegeben, die in diesem Falle gegenüber dem Mittelstück 1 exzentrisch ist.

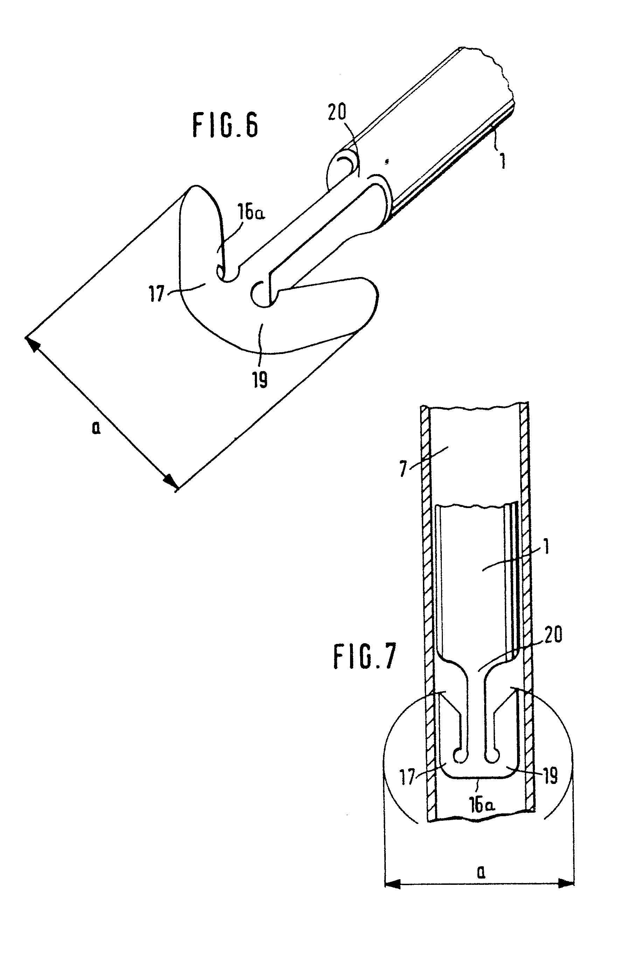

Die Fig. 6 und 7 zeigen eine weitere Ausführungsform mit einem Doppelhaken 16a. Dementsprechend zeigt dieser zwei Schwachstellen 17 und 19. In diesem Falle ist die Verbindung des Hakens 16a mit dem Mittelstück 1 zentrisch vorgesehen. In der Regel sind der Haken und das Mittelstück 1 einstückig ausgebildet.

Die Spreizung a erfolgt regelmässig nach beiden Seiten, wie man der Fig. 6 entnehmen kann.

Fig. 7 zeigt wieder das gleiche distale Ende nach Fig. 6 in seiner Einbaulage in dem Kanal 7. Die Abmessungen sind hier im übrigen die gleichen wie bei der vorangegangenen Ausführungsform.

**Patentansprüche**

1. Tubenpessar zum beidseitigen reversiblen Verschliessen des isthmischen Teils der Tube der Gebärmutter mit einem zylindrischen Mittelstück (1) aus medizinisch unwirksamen Material und Ansätzen (6b, 6a; 11a, 11b; 10, 13a, 13b; 15a, 16), an beiden Enden (3, 4) des Mittelstückes, wobei die Ansätze (6a, 6b; 10; 15a; 16) an dem distalen Ende (3) nach aussen federnd sich spreizend angeordnet sind, dadurch gekennzeichnet, dass auch das proximale Ende (4) derartige Ansätze (11a, 11b; 13a, 13b) aufweist, wobei die Ansätze beider Enden (3, 4) fadenförmig ausgebildet sind.

2. Tubenpessar nach Anspruch 1, dadurch gekennzeichnet, dass die Ansätze (11a, 11b) des proximalen Endes (4) als nach aussen federnde Schleife ausgebildet sind.

3. Tubenpessar nach Anspruch 1, dadurch gekennzeichnet, dass die Ansätze (6a, 6b) am distalen Ende (3) ebenfalls als nach aussen federnde Schleife ausgebildet sind.

4. Tubenpessar nach Anspruch 1, dadurch gekennzeichnet, dass das Mittelstück (1) eine Länge von 0,5–1,5 cm und einen Durchmesser von 1–2 mm aufweist.

5. Tubenpessar nach Anspruch 3, dadurch gekennzeichnet, dass die distale Schleife (6a, 6b) etwa oval mit einem Durchmesser von 3–6 mm ausgebildet ist.

6. Tubenpessar nach Anspruch 3, dadurch gekennzeichnet, dass die Ansätze (6a, 6b) am distalen Ende (3) einen Durchmesser aufweisen, der etwa halb so gross ist wie der Durchmesser des Mittelstückes (1).

7. Tubenpessar nach Anspruch 1, dadurch gekennzeichnet, dass das distale Ende (3) des Mittelstückes (1) weich und abgerundet ist.

8. Tubenpessar nach Anspruch 1, dadurch gekennzeichnet, dass das proximale Ende (4) des Mittelstückes (1) einen Durchmesser aufweist, der grösser ist als der des übrigen Teiles des Mittelstückes.

9. Tubenpessar nach Anspruch 1, dadurch gekennzeichnet, dass das proximale Ende (4) des Mittelstückes (1) einen Blindkanal (14) zur Einführung eines metallischen flexiblen Führungsdrahtes (15) zum Zwecke der Verankerung aufweist.

10. Tubenpessar nach Anspruch 1, dadurch gekennzeichnet, dass die Ansätze als ein oder mehrere Fäden (10, 13) ausgebildet sind, die in Richtung auf das proximale Ende (4) in einem spitzen Winkel zu dem Mittelstück (1) liegen.

11. Tubenpessar nach Anspruch 1, dadurch gekennzeichnet, dass es ein Material aufweist, das strahlungsundurchlässig ist.

12. Tubenpessar nach Anspruch 1, dadurch gekennzeichnet, dass es mit einem chemischen, metallischen oder aus einer Legierung bestehenden Mittel versehen ist, das unveränderbar bleibt.

13. Tubenpessar nach Anspruch 1, dadurch gekennzeichnet, dass die Ansätze am distalen Ende (3) als Widerhaken (15a, 16) ausgebildet sind.

14. Tubenpessar nach Anspruch 13, dadurch gekennzeichnet, dass der Widerhaken (16) exzentrisch mit dem Mittelstück (1, 18) verbunden ist und eine Schwachstelle (17) aufweist.

15. Tubenpessar nach Anspruch 13, dadurch gekennzeichnet, dass der Widerhaken als Doppelhaken (16a) ausgebildet ist, der etwa in der Mitte (20) des Mittelstückes (1) mit diesem verbunden ist und zwei Schwachstellen (17, 19) aufweist.

16. Verfahren zur Anwendung des Tubenpessars nach Anspruch 1 oder mehreren der vorangegangenen Ansprüche, bei dem das Tubenpessar mittels eines Drahtes aus Metall oder Kunststoff geführt wird, dadurch gekennzeichnet, dass das Tubenpessar in einem Kanal (7) eines Hysteroskopes (8) geführt und aus diesem herausgeschoben wird.

**Claims**

1. Tubular pessary for the reversible closure of both sides of the isthmian part of the tube of the uterus with a cylindrical central portion (1) made from medically inactive material and attachments (6b, 6a; 11a, 11b; 10, 13a, 13b; 15a, 16) at both ends (3, 4) of the central portion, the attachments (6a, 6b, 10, 15a, 16) being arranged so as to spread resiliently outwards on the distal end (3), characterized in that the proximal end (4) also has such attachments (11a, 11b, 13a, 13b), the attachments of both ends (3, 4) being constructed in filiform manner.

2. Tubular pessary according to claim 1, characterized in that the attachments (11a, 11b) of the proximal end (4) are constructed as an outwardly resilient loop.

3. Tubular pessary according to claim 1, characterized in that the attachments (6a, 6b) at the distal end (3) are also constructed as an outwardly resilient loop.

4. Tubular pessary according to claim 1, characterized in that the central portion (1) has a lenght of 0.5 to 1.5 cm and a diameter of 1 to 2 mm.

5. Tubular pessary according to claim 3, characterized in that the distal loop (6a, 6b) is approximately oval with a diameter of 3 to 6 mm.

6. Tubular pessary according to claim 3, characterized in that the attachments (6a, 6b) at the distal end (3) have a diameter which is roughly half as large as the diameter of the central portion (1).

7. Tubular pessary according to claim 1, characterized in that the distal end (3) of the central portion (1) is soft and rounded.

8. Tubular pessary according to claim 1, characterized in that the proximal end (4) of the central portion (1) has a larger diameter than the remainder of the central portion.

9. Tubular pessary according to claim 1, characterized in that the proximal end (4) of the central portion (1) has a blind channel (14) for the insertion of a metal, flexible guide wire (15) for anchoring purposes.

10. Tubular pessary according to claim 1, characterized in that the attachments are constructed as one of more filaments (10, 13), which are at an acute angle to the central portion (1) in the direction of the proximal end (4).

11. Tubular pessary according to claim 1, characterized in that it has a material which is radiopaque.

12. Tubular pessary according to claim 1, characterized in that it is provided with a chemical or metallic means or a means made from an alloy, which remains invariable.

13. Tubular pessary according to claim 1, characterized in that the attachments at the distal end (3) are constructed as barbs (15a, 16).

14. Tubular pessary according to claim 13, characterized in that the barb (16) is eccentrically connected to the central portion (1, 18) and has a weak point (17).

15. Tubular pessary according to claim 13, characterized in that the barb is constructed as a double hook (16a), which roughly in the centre (20) of central portion (1) is connected thereto and has two weak points (17, 19).

16. Method for the use of the tubular pessary according to claim 1 or more of the preceding claims, in which the tubular pessary is guided by means of a metal or plastic wire, characterized in that the tubular pessary is guided in a channel (7) of a hysteroscope (8) and is removed therefrom.

## Revendications

1. Pessaire tubulaire pour la fermeture réversible des côtés de l'isthme du tube de la matrice à l'aide d'une pièce médiane cylindrique (1) en un matériau inerte sur le plan médical et des prolongements (6b, 6a; 11a, 11b, 10, 13a, 13b; 15a, 16) aux deux extrémités (3, 4) de la pièce médiane, les prolongements (6a, 6b; 10; 15a; 16) étant prévus de manière qu'à l'extrémité distale (3) ils s'écartent élastiquement vers l'extérieur, caractérisé en ce que l'extrémité proximale (4) comporte également de tels prolongements (11a, 11b; 13a, 13b), les prolongements des deux extrémités (3, 4) étant en forme de fils.

2. Pessaire tubulaire selon la revendication 1, caractérisé en ce que les prolongements (11a, 11b) de l'extrémité proximale (4) sont réalisés sous la forme de boucles élastiques vers l'extérieur.

3. Pessaire tubulaire selon la revendication 1, caractérisé en ce que les prolongements (6a, 6b) à l'extrémité distale (3) sont également réalisés sous la forme de boucles élastiques vers l'extérieur.

4. Pessaire tubulaire selon la revendication 1, caractérisé en ce que la pièce médiane (1) a une longueur de 0,5–1,5 cm et un diamètre de 1–2 mm.

5. Pessaire tubulaire selon la revendication 3, caractérisé en ce que la boucle distale (6a, 6b) est de forme sensiblement ovale avec un diamètre de 3–6 mm.

6. Pessaire tubulaire selon la revendication 3, caractérisé en ce que les prolongements (6a, 6b) de l'extrémité distale (3) ont un diamètre qui correspond sensiblement à la moitié du diamètre de la pièce médiane (1).

7. Pessaire tubulaire selon la revendication 1, caractérisé en ce que l'extrémité distale (3) de la pièce médiane (1) est souple et arrondie.

8. Pessaire tubulaire selon la revendication 1, caractérisé en ce que l'extrémité proximale (4) de la pièce médiane (1) a un diamètre qui est supérieur à celui des autres parties des pièces médianes.

9. Pessaire tubulaire selon la revendication 1, caractérisé en ce que l'extrémité proximale (4) de la pièce médiane (1) comporte un canal aveugle (14) pour l'introduction d'un fil de guidage (15) souple, métallique, pour l'accrochage.

10. Pessaire tubulaire selon la revendication 1, caractérisé en ce que les prolongements sont réalisés sous la forme d'un ou plusieurs fils (10, 13) qui font un angle aigu par rapport à la pièce médiane (1) en direction de l'extrémité proximale (4).

11. Pessaire tubulaire selon la revendication 1, caractérisé en ce qu'il comporte un matériau perméable aux rayonnements.

12. Pessaire tubulaire selon la revendication 1, caractérisé en ce qu'il est muni d'un moyen constitué en un alliage ou en un moyen chimique, métallique qui reste invariable.

13. Pessaire tubulaire selon la revendication 1, caractérisé en ce que les prolongements de l'extrémité distale (3) sont en forme de crochets d'ancrage (15a, 16).

14. Pessaire tubulaire selon la revendication 13, caractérisé en ce que les crochets d'ancrage (16) sont reliés de manière excentrée à la pièce médiane (1, 18) et comportent un point de rupture (17).

15. Pessaire tubulaire selon la revendication 13, caractérisé en ce que le crochet d'ancrage est réalisé en forme de double crochet (16a) qui est relié à la pièce médiane (1) sensiblement en son milieu (20) et comporte deux points faibles (17, 19).

16. Procédé d'application du pessaire tubulaire selon la revendication 1 et plusieurs des revendications précédentes selon lequel le pessaire tubulaire est guidé à l'aide d'un fil métallique en matière synthétique, caractérisé en ce que le pessaire est guidé par un canal (7) d'un hystéroscope (8) et est poussé hors de celui-ci.

FIG.1

FIG.2

FIG.3

FIG.4

1

17

16

a

1

7

18

17

16

FIG.5

a

9

FIG.6

20

1

16a

17

19

a

FIG.7

7

1

20

17

19

16a

a